Europäisches Patentamt

European Patent Office

Office européen des brevets

(19)

(11) Publication number: 0 514 902 A1

# EUROPEAN PATENT APPLICATION

(12)

(21) Application number: 92108618.7

(22) Date of filing: 21.05.92

(51) Int. Cl.5: **C07C 273/02**, **B01D 53/34**

(30) Priority: 22.05.91 IT MI911413

(43) Date of publication of application:
**25.11.92 Bulletin 92/48**

(84) Designated Contracting States:
**AT BE CH DE DK ES FR GB GR IT LI LU MC NL PT SE**

(71) Applicant: **ENICHEM AGRICOLTURA S.p.A.**
**Via Ruggero Settimo, 55**
**I-90100 Palermo(IT)**

(72) Inventor: **Ognibeni, Mario, Dr.**
**Piazza Maggiore, 2**
**I-38050 Pieve, Tesino (Trento(IT)**
Inventor: **Nardon, Danilo**
**Via Arturo Chiarin, 10**
**I-30030 Campalto (Venezia)(IT)**
Inventor: **Bassan, Fabio, Dr.**
**Corso Vittorio Emanuele II, 116**
**I-35100 Padova(IT)**
Inventor: **Silvestrin, Leonardo**
**Via Don Milani, 14**
**I-30031 Dolo (Venezia)(IT)**
Inventor: **Cocquio, Fabrizio, Dr.**
**Via Alberoni 5-4**
**I-30030 Oriago di Mira (Venezia)(IT)**

(74) Representative: **von Hellfeld, Axel, Dr.**
**Dipl.-Phys. et al**
**Wuesthoff & Wuesthoff Patent- und**
**Rechtsanwälte Schweigerstrasse 2**
**W-8000 München 90(DE)**

(54) **Process for scrubbing by means of a liquid the ammonia contained in a vent.**

(57) Process for scrubbing by means of a liquid the ammonia contained in a vent (in particular the vents of plants for the synthesis and granulation of urea), wherein said liquid is an aqueous solution of urea neutralized with an acid, the pH of said neutralized solution being from 5.0 to 7.0.

_Fig. 4_

EP 0 514 902 A1

The invention relates to a process for scrubbing the ammonia contained in a vent and in particular the $NH_3$ contained in the vents of plants for the synthesis and granulation of urea, also containing air, water vapour and urea powder.

The granulation of urea is carried out, according to the most modern technologies, in rotating drums, as described, for example, in US Patent 4.686.115 and in Italian Patent 1.197.496; the invention, however, also relates to the scrubbing of polluting substances contained in vents belonging to traditional-type plants, such as the so-called prilling towers (as an example, refer to Italian Patent 1.012.772), and also to the scrubbing of polluting substances contained in other vents of different sources.

During the granulation of urea, the gas vents composed of air, urea powder and vapours are separated; these vents are usually blasted with water and the vapours, as is already known, contain a certain quantity of ammonia, which develops, almost entirely, during the transfer of the urea (in the molten state) from the final evaporator of the synthesis plant to the granulating equipment, owing to the formation of biuret. The scrubbers used up until now, generally operate at atmospheric pressure and with solutions having a temperature which ranges from room temperature to 35-40°C, thus enabling an extremely effective elimination of the powders (98% and even more, with respect to the total quantity of powders present). These aqueous solutions, however, do not effectively hold the second component to be removed i.e. ammonia; usually only 20% (approximately) of the ammonia present is removed, when washing with water is used, especially when the quantities of $NH_3$ are very small, with respect to the total mass of the gas vents. Italian Patent 1.012.772 discloses the scrubbing of urea powders (at the head of a prilling tower) with an aqueous solution of urea itself, but not even this solution is able to effectively eliminate the traces of ammonia which accompany said powders. The Applicant has now succeeded in obtaining very high scrubbing percentages of ammonia with a new procedure which is extremely simple and practical and which has given unexpected results.

In its widest aspect, the present invention relates to a process for scrubbing with a liquid the ammonia contained in a vent (and in particular the $NH_3$ contained in the vents of plants for the synthesis and granulation of urea, containing also air and urea powder), wherein the scrubbing liquid used is an aqueous solution of urea neutralized with an acid, in particular an inorganic acid, such as sulphuric acid, phosphoric acid, nitric acid and their mixtures, the pH of said neutralized solution being from 5.0 to 7.0 and preferably from 5.5 to 6.5.

Extremely high scrubbing percentages (higher than 90%, in terms of removed ammonia) are obtained when the scrubbing process is carried out in a vertical cylindrical scrubber, loaded with fillers, such as RASCHIG rings, PALL rings or BERL saddles, made of ceramics, stoneware, plastic or other material which is resistant to acids and the process conditions. To avoid stoppages, due to dirtying of the filler, it is advisable, however, to use other types of scrubber (without fillers) which will be illustrated further on.

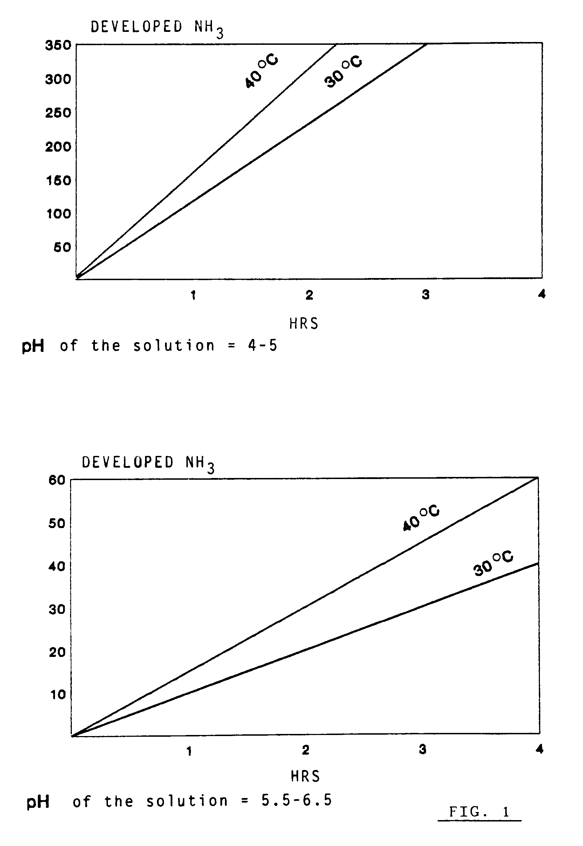

By operating within the above critical range of pH, it is possible to limit, in an extremely effective way, undesired reactions (hydrolysis of the urea to $NH_3$ and $CO_2$) triggered off and favoured by the presence of an acid. If the pH is below the lowest extreme of the range (and especially from pH 4 to pH 5, see Figure 1), the hydrolysis of the urea is uncontrollably accelerated and the quantity of $NH_3$ which develops causes considerable loss due to the fact that greater quantities of acid are required. The diagrams of Figure 1 show the development of the hydrolysis (expressed as developed $NH_3$) of an industrial solution of urea (at 17% by weight of urea), in relation to the time, at various levels of pH and temperatures. When, on the other hand, the pH level is above 7, the hydrolysis slows down, the pH does not enable effective absorption of the ammonia already present in the vent.

The concentration of the urea, in the scrubbing solutions to be used, does not particularly influence the effectiveness of the scrubbing; as a general rule, it is possible to operate with 5 to 60% by weight of urea, with respect to the scrubbing solution (preferably 10-50%). The temperature of the scrubbing solution does, on the other hand, have a considerable influence on the effectiveness; it is generally advisable to keep the thermal level of the solution within a range of from room temperature to a maximum of 40°C. The quantity of scrubbing solution depends on many factors and in particular on the capacity of the vents and concentration of the $NH_3$ contained in them; as a general rule, it is possible to use from 1 to 20 kg/hour of solution per 100 $Nm^3$/h of vent to be treated.

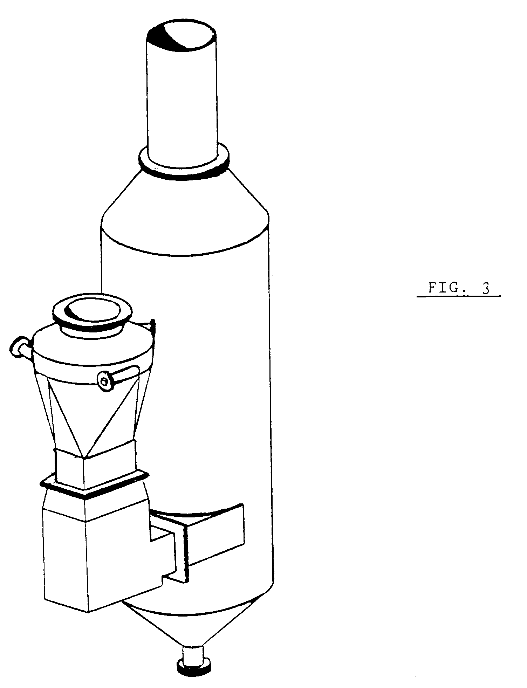

Different kinds of plants can be used for the industrial application of the present invention; Figure 2 illustrates an experimental laboratory apparatus, whereas Figure 3 shows a so-called classical "VENTURI-SCRUBBER", where a Venturi tube is connected to a cyclone by means of a flooded elbow. Figure 4 illustrates an alternative (KOCH SCRUBBER) which is very effective, even when very high charges are used; in the KOCH-type scrubber the cyclone is substituted by a squat absorption tower, with a single bubble-cap plate. The following examples provide a better illustration of the present invention but do not limit it in any way.

EXAMPLE 1 (comparative; without $H_2SO_4$

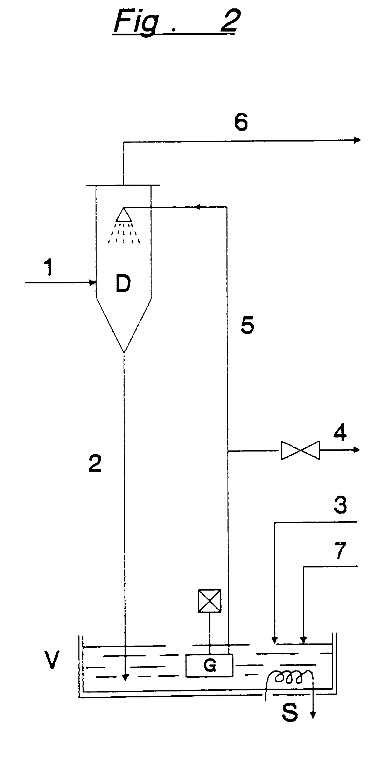

Following Figure 2, line (1) feeds (at 20°C and at 749 mmHg) an experimental gas vent (obtained artificially by mixing 1000 $Nm^3/h$ of air with 670 g/h of $NH_3$) to scrubber D, having a height of 135 cm (excluding terminal cone) and a diameter of 40 cm, over the collecting basin V (having a volume of 200 litres). The liquid (2), leaving the conical end of the scrubber, is collected in the basin and is cooled (by the coil S) and diluted with an excess of water (3) containing urea; the immersed pump G supplies the necessary energy for removing 113 Kg/h of final solution (4) and for feeding 2.9 $m^3/h$ of recycled solution (5) containing $NH_3$, urea, $(NH_4)_2SO_4$ etc. to the head of the scrubber. The purified gas vent (6) leaves the head of the scrubber D (at 12°C).

| Characteristics of the recycled solution (5): | |
|---|---|
| pH: | 10.5 |
| Urea: | 11% by weight |
| Free $NH_3$ | 1060 ppm |

| Characteristics of the purified vent (6): | |
|---|---|
| $NH_3$: | 550 g/h |
| R.H. (relative humidity): | 90% |
| Scrubbing yield: | 18%. |

EXAMPLE 2

Example 1 is repeated adding a current of sulphuric acid (7) at 98% by weight to the solution contained in collecting basin V, in such quantities as to lower the pH of the solution to a value of 6.5. The results, which are much better compared to Example 1 (yield = 41%), are shown in Table 1, where "ammoniacal N" refers to the nitrogen present as an ammonic $NH_4^+$ group and not coming from the free $NH_3$ present in the solution (i.e. not coming from $NH_4OH$).

EXAMPLE 3

Example 2 is repeated reducing the flow rate of the carrier gas (730 $Nm^3/h$), because of the higher pressure due to the assembly of Raschig rings (cylindrical cables with dimensions of 25x25) in the scrubber. The exceptional results thus obtained (yield = 96%; residuous $NH_3$ in the purified vent = 27 mg/kg) are shown in Table 1.

**TABLE 1**

| E X A M P L E | 1(*) | 2 | 3 |
|---|---|---|---|
| $H_2SO_4$ (98%) | no | yes | yes |
| pH | 10.5 | 6.5 | 6.5 |
| Air flow rate (Nm³/h) | 1000 | 1000 | 750 |
| Raschig rings | no | no | yes |
| Characteristics of the solution: | | | |
| . Urea (% by weight) | 11 | 11 | 11 |
| . Free $NH_3$ (ppm) | 1060 | 40 | 20 |
| . Ammonial N from $(NH_4)_2SO_4$ (ppm) | 0 | 2000 | 4700 |
| . $SO_4$ (ppm) | 0 | 6900 | 16000 |
| . Temperature (°C) | 8 | 10 | 12 |
| Characteristics of the clean gas vent: | | | |
| . $NH_3$ (g/h) | 550 | 394 | 27 |
| . Relative humidity (%) | 90 | 90 | 100 |
| . Yield (%) | 18 | 41 | 96 |
| (*) Comparative example | | | |

EXAMPLE 4 (comparative; without $H_2SO_4$)

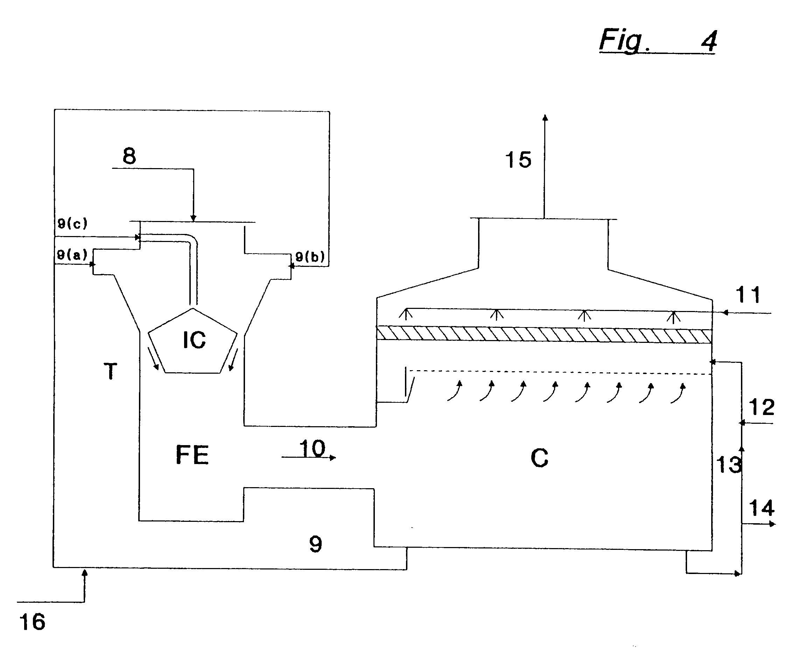

Following Figure 4, the vent (8) (429.6 t/h), coming from a plant for the granulation of urea and composed of a rotating drum, followed by a fluidized-bed cooler enters (at 70° C and at 778 mmHg) the head of a vertical T Venturi tube, with an anular section, and is accelerated in the neck, where restriction is obtained by means of an I.C. conical insert; the above vent contains 169 mg/kg of $NH_3$, together with urea powder, and at 20% of relative humidity. The solution (9), containing urea, $NH_3$ etc. is introduced into the upper section of the Venturi tube by means of tangential tubes 9(a) and 9(b), which make the liquid rotate and flow along the converging section to the neck. Part of the liquid [9(c)] is introduced by means of a centre tube at the apex of the central cone. This liquid descends along the divergent wall of the central cone to the neck; in this way, a curtain of liquid is formed against which the gas is pushed, causing its atomization. The mixture (10) thus obtained passes, through the flooded neck (FE), into a squat tower C, having a square section, containing a single bubble-cap plate, above which is a demister continually washed with condensed water. A make up of aqueous solution of urea (12), containing 17% by weight of urea, is injected into a line (13), which recycles a part of the scrubbing liquid, whose pH is 8.6, to above the bubble-cap plate (and below the demister), which accumulates on the bottom of tower C. A second part of said liquid (14) is removed from the plant, as a final product, and a third part (9) is recycled to the Venturi (T) tube. The washed gas vent (15) leaves the head of tower C (at 30°C and 92% of relative humidity), with a content of $NH_3$ equal to 120 mg/kg. The scrubbing yield is therefore very low (29%); data and results are shown in Table 2.

EXAMPLE 5

Example 4 is repeated applying some minor variations and injecting 155 Kg/h of $H_2SO_4$ (at 98% by weight; see line 16) into the line (9), thus lowering the pH of the recycled solution to a value of 5.5. Here are a few details: 473.5 t/h of a gas vent (8) entered (at 69°C and at 777 mmHg) the head of the Venturi tube; this vent contained 146 mg/kg of $NH_3$ (together with urea powder) and had a relative humidity of 17%. After mixing with the solution (9), having a pH of 5.5, the mixture passed into tower C, operating as described in Example 4. The purified gas vent (15) on leaving the head of the tower (at 31°C and at a relative humidity of 93%) contained 31.5 mg/kg of $NH_3$, which represents an extremely high yield (on an industrial scale) i.e. equal to 78.4%. Data and results are shown in Table 2. Line (14) recycled the solution produced to the plant.

At the end of the test the end product is characterized under a chemical and physical profile.

The commercial title was amply reached and the urea contained about 3400 ppm of $(NH_4)_2SO_4$; the results are shown in Table 3.

EP 0 514 902 A1

**TABLE 2**

| E X A M P L E | 4(*) | 5 |
|---|---|---|
| $H_2SO_4$ | no | yes |
| pH | 8.6 | 5.5 |
| Flow rate of the inlet gas vent (Kg/h) | 429,572 | 473,487 |
| Characteristics of the solution: | | |
| . Urea (% by weight) | 43 | 44 |
| . Free $NH_3$ (ppm) | 2680 | 15 |
| . Ammonial N from $(NH_4)_2SO_4$ (ppm) | 0 | 5690 |
| . $SO_4$ (ppm) | 0 | 19520 |
| . Temperature (°C) | 28 | 29 |
| . Flow rate (Kg/h) | 7850 | 7850 |
| Characteristics of the clean gas vent: | | |
| . $NH_3$ (Kg/h) | 51.5 | 14.9 |
| . Relative humidity (%) | 92 | 93 |
| . Yield (%) | 29.0 | 78.4 |
| (*) Comparative example | | |

TABLE 3

| | | |
|---|---|---|
| Ureic N | (%) | 46.3 |
| Ammonial N | (ppm) | 750 |
| Humidity ($H_2O$) | (%) | 0.20 |
| Free $NH_3$ | (ppm) | 109 |
| Formaldehyde | (%) | 0.35 |
| Biuret | (%) | 1.12 |
| pH | - | 8.20 |
| $SO_4^{--}$ | (ppm) | 2580 |
| Breaking load (on pellets with a diameter of 2.5-3.0 mm) | (kg) | 2.1/2.8 |
| Fine powder | (ppm) | 250 |
| Packing behaviour | | free flowing |
| Critical relative humidity (UCR or CRH) at 30°C | (%) | 64 |
| PARTICLE SIZE | | |
| more than 5 mm | (%) | 1.0 |
| 5 - 4 mm | (%) | 21.1 |
| 4 - 3 mm | (%) | 49.2 |
| 3 - 2 mm | (%) | 26.5 |
| 2 - 1 mm | (%) | 2.2 |
| less than 1 mm | (%) | 0 |

Claims

1. Process for scrubbing with a liquid the ammonia contained in a gas vent, and in particular the $NH_3$ contained in the vents of plants for the synthesis and granulation of urea, also containing air and urea powder, wherein the scrubbing liquid used is an aqueous solution of urea neutralized with an acid, in particular with an inorganic acid, such as sulphuric acid, phosphoric acid, nitric acid and their mixtures, the pH of said neutralized solution being from 5.0 to 7.0 and preferably from 5.5 to 6.5.

2. The process according to Claim 1, wherein the concentration of urea, in the scrubbing liquid, is from 5 to 60% by weight (preferably from 10 to 50%).

3. The process according to any of the previous Claims, wherein the temperature level is kept within a range from room temperature to 40°C.

7

4. The process according to any of the previous Claims, wherein the quantitay of scrubbing liquid is from 1 to 20 kg/hour every 100 Nm$^3$ of gas vent.

5. The process according to any of the previous Claims, wherein the scrubbing is carried out in a vertical cylindrical scrubber, preferably loaded with fillers, in particular RASCHIG rings.

6. The process according to any of the previous Claims, wherein the scrubbing is carried out in an apparatus composed of a Venturi tube connected, by means of a flooded elbow, to an absorption tower containing at least one bubble-cap plate.

FIG. 1

# _Fig . 2_

FIG. 3

EP 0 514 902 A1

_Fig._ 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | DE-A-3 712 494 (M.E. IVANOV ET AL.)<br>* column 13, line 44 - line 52; example 3 *<br>--- | 1-3 | C07C273/02<br>B01D53/34 |
| A | EP-A-0 084 669 (NORSK HYDRO)<br>* page 8; claims 1-3 *<br>* page 5 - page 7, example 2; especially page 6, paragraph 2 and page 7, paragraph 2 *<br>--- | 1-6 | |
| A | US-A-4 424 072 (B.J. LERNER)<br>* column 8, line 20 - line 48 *<br>* column 10, line 13 - line 18 *<br>----- | 1 | |

| | | | TECHNICAL FIELDS SEARCHED (Int. Cl.5 ) |
|---|---|---|---|
| | | | C07C<br>B01D |

The present search report has been drawn up for all claims

| Place of search | Date of completion of the search | Examiner |
|---|---|---|
| THE HAGUE | 24 AUGUST 1992 | FINK D.G. |